# EUROPEAN PATENT APPLICATION

(11) **EP 2 410 333 A1**
(43) Date of publication of application: **25.01.2012**
(21) Application number: 11174981.8
(22) Date of filing: 22.07.2011
(51) Int. Cl.: G01N 33/53

(54) **Method of detecting target, method of suppressing increase in background and detection apparatus**

(30) Priority: 23.07.2010 JP 2010166413; 07.07.2011 JP 2011151092
(71) Applicant: Arkray, Inc., Minami-ku Kyoto-shi Kyoto 601-8045 (JP)
(72) Inventor: Onuma, Naotsugu, Kyoto-shi Kyoto, 602-0008 (JP); Takagi, Mikako, Kyoto-shi Kyoto, 602-0008 (JP)
(74) Representative: Golding, Louise Ann

(57) **Abstract**

Provided are a method of detecting a target with high accuracy, a method of suppressing an increase in background, and a detection apparatus.

A first target-binding substance that is immobilized at a carrier and is capable of binding to a target and a second target-binding substance that supports a labeling substance and is capable of binding to the target are used. By reacting a sample, the first target-binding substance, and the second target-binding substance, a complex of the target in the sample, the first target-binding substance, and the second target-binding substance is formed in a first container. Subsequently, after the complex is transferred from the first container to a second container, the complex is detected by detecting the labeling substance in the second container.

## Description

### BACKGROUND OF THE INVENTION

The present invention relates to a method of detecting a target, a method of suppressing an increase in background, and a detection apparatus.

Heretofore, a binding assay has been used widely as a method of performing a qualitative analysis or a quantitative analysis of a target in a sample. The binding assay uses two types of binding substances capable of binding to a target, for example. Generally, the binding substances are, for example, antibodies to a target. In this method, one of the antibodies is immobilized at a carrier such as a magnetic particle (hereinafter, referred to as the "immobilized antibody") and the other of the antibodies is labeled with a labeling substance such as an enzyme (hereinafter, referred to as the "labeled antibody"). Further, in a reaction system, the target, the immobilized antibody, and the labeled antibody are reacted. Thereby, the immobilized antibody and the labeled antibody are bound to the target, and the complex thereof is formed. However, the reaction system contains an unreacted liberated labeled antibody that is not involved in the formation of the complex. Hence, in the reaction system, the complex containing the labeled antibody is separated from the liberated unreacted labeled antibody by the B (Bond) / F (Free) separation. Thereby, the signal of the labeled antibody of the complex can be detected without being affected by the liberated unreacted labeled antibody. Since there is a correlation between the amount of the labeled antibody in the complex and the amount of the target in the complex, the target can be analyzed based on the signal (see, for example, JP H8(1996)-29424 A).

### BRIEF SUMMARY OF THE INVENTION

The present invention is intended to provide a method of detecting a target with high accuracy, a method of suppressing an increase in background, and a detection apparatus.

The method of detecting a target of the present invention uses a first target-binding substance immobilized at a carrier and a second target-binding substance labeled with a labeling substance. The method includes the steps of reacting a target, the first target-binding substance, and the second target-binding substance to form a complex of the target, the first target-binding substance, and the second target-binding substance in a first container; transferring the complex from the first container to a second container; and detecting the complex by detecting the labeling substance in the second container.

The method of suppressing an increase in background in detection of a target uses a first target-binding substance immobilized at a carrier and a second target-binding substance labeled with a labeling substance. The method includes the steps of reacting a target, the first target-binding substance, and the second target-binding substance to form a complex of the target, the first target-binding substance, and the second target-binding substance in a first container; transferring the complex from the first container to a second container; and detecting the complex by detecting the labeling substance in the second container.

The detection apparatus of the present invention is used for the method of detecting a target of the present invention. The detection apparatus includes a first container, a second container, a transfer unit, and a detection unit. The first container is the first container used in the method of detecting a target of the present invention, the second container is the second container used in the method of detecting a target of the present invention, the transfer unit is a unit for transferring the complex from the first container to the second container, and the detection unit is a unit for detecting the labeling substance in the step of detecting the complex.

In the aforementioned conventional binding assay, when the labeled binding substance is nonspecifically bound to a substance other than the target, a signal due to the nonspecific binding occurs. Therefore, for example, the background is increased, and the accuracy of analysis is decreased. Accordingly, suppression of the occurrence of signal due to the nonspecific binding is an important issue of the accuracy of analysis. It is predicted that such a problem is caused, for example, by a nonspecific binding of the labeled antibody to the carrier such as the magnetic particle. Hence, in order to suppress the nonspecific binding to the carrier, in the binding assay, for example, treatment of the surface of the carrier with a blocking agent such as bovine serum albumin, casein, or gelatin; addition of a surfactant to the reaction system; and use of an antibody from which a Fc region is removed are attempted. However, even by these methods, a problem remains that a sufficient accuracy of analysis cannot be obtained due to the unreacted labeled antibody. There is currentry a demand for a further increase in accuracy. Therefore, it is desirable to suppress the effect of the unreacted labeled antibody and to establish a method of further improving the accuracy of analysis. This problem is not limited to the antibody and can also be an issue with regard to other binding substances capable of binding to a target in the binding assay.

As a result of keen studies made assiduously, the inventors of the present invention discovered that the accuracy of analysis was decreased due to the nonspecific adsorption of the unreacted labeled binding substance that was not involved in the formation of the complex among the labeled binding substances to a container. In other words, as with the labeled binding substance in the complex, the unreacted labeled binding substance that is nonspecifically adsorbed to the container is also detected, and thereby a background is increased and the accuracy of analysis is decreased. Hence, it was discovered that when the complex formed was transferred from the container that was used for the formation of the complex to another container and detection of the labeled binding substance was performed in the container to which the complex is transferred, the detection was not affected by the labeled binding substance that was nonspecifically adsorbed to the container that was used for the formation of the complex. Therefore, according to the present invention, for example, the occurrence of a signal caused by the labeled binding substance that is not involved in the formation of the complex and adsorbed to the container can be suppressed and thereby an increase in the background can be suppressed. Thus, according to the present invention, for example, the target can be detected with high accuracy through the detection of the labeled binding substance. The method of the present invention is very useful, for example, for research in medicine, biochemistry, and the like and tests of clinical medical cares, foods, environments, and the like.

### DETAILED DESCRIPRION OF THE INVENTION

### <Detection Method of Target>

As described above, the detection method of the present invention uses a first target-binding substance immobilized at a carrier and a second target-binding substance labeled with a labeling substance. The method includes the steps of reacting a target, the first target-binding substance, and the second target-binding substance to form a complex of a target, the first target-binding substance, and the second target-binding substance in a first container (complex formation step); transferring the complex from the first container to a second container (complex transfer step); and detecting the complex by detecting the labeling substance in the second container (complex detection step).

Hereinafter, the first target-binding substance immobilized at the carrier is also referred to as the "first immobilized substance" and the second target-binding substance labeled with the labeling substance is also referred to as the "second labeled substance".

In the present invention, the second target-binding substance that is not involved in the formation of the complex is also referred to as the "unreacted second target-binding substance". Among the unreacted second target-binding substances, the one that is not adsorbed to the first container is also referred to as the "liberated second target-binding substance" and the one that is adsorbed to the first container is also referred to as the "adsorbed second target-binding substance".

The method of detecting a target of the present invention is characterized in that the complex formed in the first container is transferred to a new second container and detection of the complex by the detecting the labeling substance is performed in the second container, and steps except for these are not limited at all.

In the detection method of the present invention, the complex detection step is preferably a step of reacting a detection reagent capable of detecting the labeling substance and the complex to detect the labeling substance through detection of the reaction in the second container, for example.

The shapes of the first container and the second container are not particularly limited. Each container can be any shape as long as components can coexist in the complex formation step and the complex detection step, for example. The container may be, for example, a tubular container or a chamber formed on a base plate. The tubular container may be a container with a bottom or a container without a bottom, for example. Examples of the tubular container with a bottom include a micro tube, a test tube, a cuvette, and a sample cup. For example, the tubular container without a bottom may be a chip for micropipetting. For example, the chamber may be a concaved portion formed on a base plate. Specifically, examples thereof include chambers formed on devices such as a well of a well plate, a micro TAS (µTAS), and a microchip. In each of those devices, for example, the chamber may be in communication with a channel or may be a part of the channel.

The material of the container is not limited. Examples of the material include polymer materials such as inorganic polymers and organic polymers. Examples of the inorganic polymers include, but are not limited to, synthetic fused silica, molten silica, and borosilicate glass. Examples of the organic polymers include, but are not limited to, polypropylene, polyethylene, polystyrene, polytetrafluoroethylene, polymethyl methacrylate, cycloolefin polymer, polycarbonate, polydimethylsiloxane, polylactic acid, polyether ether ketone, and polyethylene terephthalate.

The first target-binding substance and the second target-binding substance are not particularly limited as long as they are capable of binding to the target. Examples of the first target-binding substance and the second target-binding substance include antibodies, antigens, and nucleic acids; and the first target-binding substance and the second target-binding substance are preferably antibodies. For example, the types of the first target-binding substance and the second target-binding substance may be identical to each other or different from each other. Preferably, the first target-binding substance and the second target-binding substance bind to the target at different sites, for example. In the case where both of the first target-binding substance and the second target-binding substance are antibodies, preferably, the different sites of the target are epitopes.

The antibodies are not particularly limited as long as they are capable of binding to the target. The types of the antibodies are not particularly limited and examples thereof include immunoglobulin molecules such as IgG, IgA, IgM, IgD, IgE, and IgY; and antibody fragments thereof such as Fab, Fab', F(ab')₂, a heavy-chain variable region (V_{H}), and a light chain variable region (V_{L}). The antibodies may be, for example, antibodies derived from animal species such as human and nonhuman mammals such as mice, rabbits, cattle, pigs, horses, sheep, and goats; and birds such as chickens. The antibodies may be prepared, for example, from blood serum derived from the animal species by a conventional method. Also, commercially available antibodies may be used. The antibodies may be, for example, polyclonal antibodies or monoclonal antibodies. Hereinafter, in the present invention, the "antibody" includes an antibody fragment unless otherwise noted.

The antigens are not particularly limited as long as they are capable of binding to the target. In the case where the first target-binding substance is an antigen, examples of the target include an antibody to the antigen and an antibody fragment.

The nucleic acids are not particularly limited as long as they are capable of binding to the target. The types of the nucleic acids are not particularly limited and examples thereof include DNA and RNA.

As described above, the first target-binding substance and the second target-binding substance can be any substances as long as they are capable of binding to the target, and the combination thereof is not particularly limited. Specific examples of the combination are described below. However, the present invention is not limited thereto. In the case where the target is an antigen, for example, the both binding substances are antibodies to the target (antigen). In the case where the target is an antibody, one of the binding substances is an antigen to which the target (antibody) is bound and the other of the binding substances is an antibody capable of binding to the target (antibody). Further, for example, both of the first target-binding substance and the second target-binding substance may be nucleic acids or one of them may be the nucleic acid and the other may be an antigen or an antibody to the target.

As described above, the first target-binding substance can be any substance as long as it is capable of binding to the target. Other than the above, the first target-binding substance may be at least one selected from the group consisting of biotin, avidin, streptavidin, proteins, peptide, sugar chains, lipid, hormones, ligand, and receptors. For example, in the case where the target is biotin, the first target-binding substance is avidin or streptavidin and in the case where the target is avidin or streptavidin, the first target-binding substance is biotin.

The carrier that immobilizes the first target-binding substance is not particularly limited. The carrier can be any carrier as long as it is capable of immobilizing the first target-binding substance, for example. For example, the carrier may be the one that is insoluble to a liquid reaction system and can be recovered. Specifically, examples thereof include, but are not limited to, particles, sponges, reticulated structures, and membranes. Examples of the particles include metal colloid particles such as magnetic particles, latex particles, gold colloid particles, and platinum colloid particles; polymer particles such as polymethyl methacrylate particles and polylactic acid particles; glass particles; alumina particles; silica gel particles; and activated carbons. The average particle size of the particles is not particularly limited and can be set appropriately depending on the type of particle, for example. Other than the aforementioned particles, examples of the carrier include DNAs, linear polymers, and dendrimers.

The method of immobilizing the first target-binding substance to the carrier is not particularly limited, and a conventionally known method can be employed. For example, the first target-binding substance may directly be immobilized at the carrier or indirectly be immobilized at the carrier. In the latter case, examples of the linker that binds the carrier and the first target-binding substance include, but are not limited to, avidin, streptavidin, biotin, glutaraldehyde, and albumin. Specifically, for example, the first target-binding substance may be immobilized at the carrier through avidin or streptavidin and biotin. In this case, for example, the avidin or the streptavidin may be bonded to the carrier and the biotin may be bonded to the first target-binding substance. The first target-binding substance can be immobilized at the carrier through a binding between the avidin or the streptavidin and the biotin.

The labeling substance with which the second target-binding substance is labeled is not particularly limited. For example, the labeling substance may be a substance that can be detected alone or a substance that can be detected through reaction with the detection reagent. Examples of the labeling substance include chemiluminescent substances such as enzymes, acridinium esters, and luminol; fluorescent substances such as fluorescein isothiocyanate (FITC); and DNA. The enzymes are not particularly limited and examples thereof include alkaline phosphatase, peroxidase, and 6-D-galactosidase.

The method of labeling the second target-binding substance with the labeling substance is not particularly limited and a conventionally known method can be employed.

The object of the present invention is to suppress the effect of the unreacted second labeled substance that is not involved in the formation of the complex and adsorbed to the container; and the target is not particularly limited. Examples of the target include pathogens, biogenic substances, endocrine-disrupting chemicals, allergic substances in foods, and agricultural chemicals. Specific examples include, but are not limited to, pathogen antigens such as influenza A virus, influenza B virus, influenza C virus, adenovirus, RS virus, coronavirus, astrovirus, norovirus, measles virus, rotavirus, human immunodeficiency virus (HIV), human T-cell leukemia virus (HTLV-1), hepatitis B virus (HBV), hepatitis C virus (HCV), herpesvirus, mycoplasma, Treponema pallidum, Chlamydia trachomatis, tubercle bacillus, coliform, Group A Streptococcus, Group B Streptococcus, Streptococcus pneumoniae, Staphylococcus, MRSA, Legionella, enterohemorrhagic Escherichia coli 0157, verotoxin, Salmonella, Clostridium difficile, and Helicobacter pylori; diagnostic markers such as CRP, HBs, HBc, HBe, prostate-specific antigen (PSA), troponin T, troponin I, myoglobin, D-dimer, fecal hemoglobin, hemoglobin Alc, and IgE antibody; hormones such as human chorionic gonadotrophin (hCG) and luteinizing hormone (LH), insulin, and C-peptide; steroid hormones such as thyroxine, cortisol, and estradiol; endocrine-disrupting chemicals such as bisphenol A, nonylphenol, and 4-octylphenol; allergens contained in wheat, buckwheat, peanuts, eggs, shrimps, crabs, milk, and the like; and nucleic acids.

The samples to which the method of detecting a target of the present invention is applied are not particularly limited. Specific examples of the samples include biological samples such as whole blood, blood serum, blood plasma, sweat, urine, nasal aspirates, nasal lavage fluids, nasal swabs, nasal discharges, throat swabs, gargled fluid, saliva, cells, and feces; water of seas, lakes, and rivers; soils; and foods. For example, the samples may be extracts, dissolved substances, dispersed substances, suspended substances, and the like that are prepared using solvents. Examples of the solvents include, but are not limited to, water, physiological saline, and buffer solutions. Examples of the buffer solutions include, but not limited to, Tris buffers, phosphate buffers, acetate buffers, and borate buffers. For example, the solvent may further contain a surfactant, a stabilizer, an antimicrobial agent, and the like.

Next, embodiments of the detection method of the present invention will be described in detail. However, the detection method of the present invention is not limited to the following embodiments.

### (Embodiment 1)

The detection method of this embodiment includes the complex formation step, the complex transfer step, and the complex detection step.

In the complex formation step, first, the sample, the first immobilized substance, and the second labeled substance are reacted to form a complex of the target, the first immobilized substance, and the second labeled substance in the first container. The reaction system that forms the complex is preferably, for example, a liquid reaction system (reaction solution).

In the complex formation step, it is applicable as long as the sample, the first immobilized substance, and the second labeled substance eventually coexist in the first container. The first immobilized substance and the second labeled substance can be added to the first container at the time of using, for example. In this case, the order of adding the first immobilized substance, the second labeled substance, and the sample is not particularly limited. Specifically, for example, the first immobilized substance, the second labeled substance, and the sample may be added separately, two of them may be added at the same time, or three of them may be added at the same time. One of the first immobilized substance and the second labeled substance or both of them may be arranged preliminarily in the first container, for example.

In the case where the first immobilized substance and the second labeled substance are added to the first container, for example, each of them may be added after being mixed with a solvent. Examples of the solvent include, but not limited to, the aforementioned solvents.

Conditions for forming the complex are not particularly limited. Specifically, for example, the sample, the first immobilized substance, and the second labeled substance are preferably incubated at 2 to 60°C for 1 to 60 minutes, such as at 20 to 40°C for 5 to 15 minutes.

Next, in the complex transfer step, the complex is transferred from the first container to the second container. As described above, the inventors of the present invention found out that the second labeled substance is nonspecifically adsorbed to the container in which the sample, the first immobilized substance, and the second labeled substance are reacted, and the accuracy of analysis is thereby decreased. Therefore, by transferring the complex from the first container in which the complex formation step is performed to the new second container in this manner, the effect of the nonspecific adsorption of the second labeled substance to the first container to the accuracy of analysis can be avoided.

The transfer method is not particularly limited, and can be decided appropriately depending on the type of carrier. For example, the complex may be transferred by sucking it from the first container and discharging it to the second container. Further, in the case where the carrier is a magnetic particle, for example, the complex may be transferred using a magnetic body having a magnetic force such as a magnet. In the case where the carrier is DNA, for example, the complex may be transferred by an electrophoresis. The transfer unit that will be described below can be used for the transfer, for example.

Further, in the complex detection step, the labeling substance is detected in the second container. The complex can be detected through the detection of the labeling substance, and as a result, the target that has been involved in the formation of the complex can be detected.

With respect to the detection of the labeling substance, for example, a method suitable to the labeling substance can be employed appropriately

In the case where the labeling substance is a substance that can be detected alone, for example, the signal of the labeling substance is detected. Specifically, for example, the coloring (color change and color development), the fluorescence, and the like of the labeling substance can be detected as a signal. For example, the intensity of the signal can be measured as a transmittance, an absorbance, a reflectance, a luminescence intensity, a fluorescence intensity, and the like. Conditions for the detection are not particularly limited, and can be set depending on the type of labeling substance, for example. In the case where the labeling substance is the fluorescent substance, for example, a reaction system containing the complex is irradiated with ultraviolet light, and the excitation light thereof can be detected as a signal. For example, the detection of the labeling substance may be performed in the second container or the detection may be performed after the reaction system containing the complex is transferred from the second container to a new measurement container. The detection unit that will be described below can be used for the detection, for example.

In the case where the labeling substance is a substance that can be detected through reaction with a detection reagent, for example, the detection reagent capable of detecting the labeling substance and the complex are reacted, and the reaction of the detection reagent is detected.

Specifically, the reaction is a reaction between the detection reagent and the second labeled substance of the complex. Through the detection of this reaction, the complex can be detected, and as a result, the target that has been involved in the formation of the complex can be detected.

The method of detecting the reaction is not particularly limited, and a method suitable to the labeling substance can be employed appropriately, for example. In the case where the labeling substance is an enzyme, for example, the signal of the product that is generated by the reaction of the enzyme and the detection reagent or the signal of the detection reagent that is quenched by the reaction of the enzyme and the detection reagent is detected. Specifically, for example, the coloring (color change and color development), the fluorescence, and the like of the product can be detected as a signal; and the coloring (color change and color development), the fluorescence, and the like of the detection reagent that is quenched by the reaction can be detected as a signal. For example, the intensity of the signal can be measured as a transmittance, an absorbance, a reflectance, fluorescence intensity, and the like. Conditions for the detection are not particularly limited, and can be set appropriately depending on the type of enzyme and detection reagent, for example. In the case where the labeling substance is the chemiluminescent substance, for example, the signal of the chemiluminescent substance is detected. Specifically, for example, the luminescence, the fluorescence, and the like of the product can be detected as a signal. For example, the intensity of the signal can be measured as luminescence intensity, fluorescence intensity, and the like. In the case where the labeling substance is DNA, for example, the signal of the product that is generated by the reaction of the DNA and the detection reagent is detected. Specifically, for example, the luminescence, the fluorescence, and the like of the product can be detected as a signal. For example, the intensity of the signal can be measured as luminescence intensity, fluorescence intensity, and the like.

For example, the detection of the reaction of the detection reagent may be performed in the second container or the detection may be performed after the reaction system of the reaction is transferred from the second container to a new measurement container. The detection unit that will be described below can be used for the detection, for example.

The detection reagent can be any detection reagent as long as it is capable of detecting the labeling substance; and the detection reagent can be selected appropriately depending on the labeling substance, for example. Examples of the detection reagent include, but are not limited to, a substrate that generates a product that emits a signal by the reaction, and a substrate whose signal is quenched by the reaction. In the case where the labeling substance is the enzyme, the detection reagent can be selected appropriately depending on the type of enzyme, for example. Specific examples thereof include, but are not limited to, 4-methylumbelliferyl phosphate (4-MUP), 5-bromo-4-chloro-3-indolyl phosphate (BCIP), 2,2'-azino-bis(3-ethylbenzothiazoline-6-sulfonic acid) (ABTS), 3,3',5,5'-tetramethylbenzidine (TMB), diaminobenzidine (DAB), 4-methylumbelliphenyl-6-D-galactoside (4-MUG), 3-(2'-spiroadamantane)-4-methoxy-4-(3"-β-D-galactopyranosyl)phenyl-1, and 2-dioxetane (AMGPD). In the case where the labeling substance is the chemiluminescent substance, the detection reagent can be selected appropriately depending on the type of chemiluminescent substance, for example. A specific example thereof includes hydrogen peroxide. In the case where the labeling substance is DNA, an example of the detection reagent includes 3,4,5-trimethoxyphenylglyoxal (TMPG).

In the complex detection step, the reaction of the detection reagent can be performed by allowing the complex and the detection reagent to coexist eventually in the second container, for example. The detection reagent may be added to the second container at the time of using or may be arranged preliminarily in the second container, for example. In the former case, the order of adding the complex and the detection reagent is not particularly limited. Specifically, for example, the complex and the detection reagent may be added separately or both of them may be added at the same time.

Reaction conditions of the detection reagent are not particularly limited. For example, the reaction conditions can be set appropriately depending on the type of labeling substance. In the case where the labeling substance is the enzyme, for example, the complex and the detection reagent are preferably incubated at 2 to 60°C for 1 to 60 minutes, such as at 20 to 40°C for 5 to 15 minutes.

### (Embodiment 2)

In addition to the complex formation step, the complex transfer step, and the complex detection step, the detection method of this embodiment further includes a removal step. Specifically, the detection method includes the removal step after the complex formation step; and the complex transfer step is performed after the removal step. This embodiment can be performed in the same manner as in embodiment 1 unless otherwise noted.

In this embodiment, in the same manner as in embodiment 1, after the complex is formed in the first container, the liberated second labeled substance is removed from the first container. The liberated second labeled substance is, for example, the one that is not involved in the formation of the complex and is not adsorbed to the first container as described above.

For example, the liberated second labeled substance can be removed from the first container after the liberated second labeled substance is separated from the complex. The methods of separating and removing the liberated second labeled substance are not particularly limited and conventionally known methods can be employed depending on the type of carrier used, for example. In the case where the carrier is a magnetic particle, for example, a liquid component of the reaction system containing the liberated second labeled substance may be removed in a state where the complex is captured. Specifically, for example, the complex may be captured using the magnetic body such as a magnet and the liquid component may be removed using a sucking-and-discharging unit. In the case where the magnetic body is used, for example, by bringing the magnetic body close to the external wall of the container, the complex can be captured via the external wall, and by bringing the magnetic body away from the external wall, the complex that has been captured via the external wall can be released. At the time of capturing the complex, the magnetic body is preferably brought into contact with the external wall of the container. In the case where the carrier is a latex particle, a metal colloid particle, a polymer particle, or a glass particle, for example, the liquid component (supernatant) of the reaction system containing the liberated second labeled substance may be removed while the complex is precipitated. Specifically, for example, the liquid component may be removed using the sucking-and-discharging unit while the complex is precipitated by a centrifugal force. The sucking-and-discharging unit will be described below. In this manner, for example, the unreacted liberated second labeled substance that exists in the liquid component can be removed by removing the liquid component.

Further, after the removal step, in the same manner as in embodiment 1, the complex transfer step and the complex detection step are performed. For example, after the liquid component is removed in the removal step, a solvent may be added to the first container, the solvent and the complex that has been captured may be mixed, and the resultant mixture may be transferred to the second container.

Further, in the case where the carrier is a magnetic particle, for example, the complex can be transferred from the first container to the second container by capturing the complex by the magnetic body such as a magnet and by transferring the magnetic body. In this case, preferably, the first container is in communication with the second container via a channel, for example. An example of such a device includes the aforementioned µTAS. In the device, for example, the complex is captured by arranging the magnetic body at the outside of the device and by bringing the magnetic body close to the external wall of the first container, and the magnetic body is further transferred from the external wall of the first container to the external wall of the second container via the external wall of the channel. Thereby, the complex can be transferred from the first container to the second container via the cannel.

Further, in the case where the carrier is DNA, for example, the complex can be transferred by an electrophoresis. In this case, for example, preferably, the first container is in communication with the second container via the channel, and more preferably, two regions that are located far from each other in the flow direction in the channel are the first container (first chamber) and the second container (second chamber). An example of such a device includes the aforementioned µTAS. The device can transfer the complex by an electrophoresis by applying the voltage between the first container and the second container, for example.

### (Embodiment 3)

The removal step of the embodiment 2 may be performed, for example, after the complex transfer step and before the complex detection step. The detection method of this embodiment includes the complex formation step, the complex transfer step, the removal step, and the complex detection step. Specifically, the detection method includes the removal step after the complex transfer step, and the complex detection step is performed after the removal step. This embodiment can be performed in the same manner as in embodiments 1 and 2 unless otherwise noted.

In this embodiment, in the same manner as in embodiment 1, the complex is formed in the first container and the complex is transferred to the second container. Next, in the same manner as in embodiment 2, the liberated second labeled substance that is not involved in the formation of the complex is separated from the complex, and then the liberated second labeled substance is removed from the second container. Further, the complex detection step is performed after the removal step in the same manner as in embodiment 1.

### (Embodiment 4)

The detection method of this embodiment includes the complex formation step, the removal step, the complex transfer step, and the complex detection step. The removal step further includes a washing step of washing the complex. This embodiment can be performed in the same manner as in embodiments 2 and 3 unless otherwise noted.

In this embodiment, the removal step may be performed after the complex formation step and before the complex transfer step or may be performed after the complex transfer step and before the complex detection step. Further, the removal step may be performed after the complex formation step and before the complex transfer step and also performed after the complex transfer step and before the complex detection step.

In this embodiment, in the same manner as in embodiment 2, first, the complex is formed in the first container, and then a liquid component of the reaction system containing the liberated second labeled substance is removed in a state where the complex is captured.

Then, the complex is washed. The washing method is not particularly limited, and a conventionally known method can be employed depending on the type of carrier used, for example. The washing of the complex can be performed, for example, in the first container. Specifically, for example, the capture of the complex is released and the complex is washed with a washing liquid in the first container. At this time, for example, the washing liquid may be added to the first container before the capture of the complex is released or the washing liquid may be added to the first container after the capture of the complex is released. Also, the complex may be washed in the first container in the state where the complex is captured. Then, for example, in the same manner as described above, the washing liquid in the first container is removed in the state where the complex is captured. Thereby, for example, the liberated second labeled substance that is nonspecifically bound to the complex can be removed and an analysis with higher accuracy can be achieved.

The washing step may be performed, for example, once or may be repeated several times.

The washing liquid is not particularly limited, and examples thereof include water, physiological saline, and buffer solutions. Examples of the buffer solutions include the aforementioned buffer solutions. For example, a surfactant, a stabilizer, an antimicrobial agent, or the like may be added to the washing liquid.

After the removal step, in the same manner as in embodiment 2, the complex transfer step and the complex detection step are performed.

Further, in this embodiment, in the same manner as in embodiment 3, the complex formation step, the complex transfer step, the removal step, and the complex detection step are performed. As described above, the washing step may be performed in the removal step. In this case, the washing of the complex can be performed, for example, in the second container.

### <Method of Suppressing Increase in Background >

As described above, the method of suppressing an increase in background in detection of a target uses a first target-binding substance immobilized at a carrier and a second target-binding substance labeled with a labeling substance. The method includes the steps of reacting a target, the first target-binding substance, and the second target-binding substance to form a complex of the target, the first target-binding substance, and the second target-binding substance in a first container; transferring the complex from the first container to a second container; and detecting the complex by detecting the labeling substance in the second container.

In the present invention, for example, the background refers to a factor other than the labeling substance added to the second labeled substance in the complex. For example, the background refers to a detection value caused by the labeling substance in the unreacted liberated second labeled substance that is not involved in the formation of the complex. Therefore, in the present invention, the suppression of an increase in background means, for example, the suppression of detection of the factor and the suppression of an increase in the detection value caused by the factor or the reduction of the detection value caused by the factor.

The method of suppressing an increase in background of the present invention is, for example, the same as the detection method of the present invention. Specifically, for example, all of the steps and the conditions thereof are the same as those of the detection method of the present invention.

In the method of suppressing an increase in background of the present invention, the complex detection step is preferably a step of reacting a detection reagent capable of detecting the labeling substance and the complex to detect the labeling substance through detection of the reaction in the second container.

### <Detection Apparatus>

As described above, the detection apparatus of the present invention is used for the method of detecting a target of the present invention. The detection apparatus includes a first container, a second container, a transfer unit, and a detection unit. The first container is the first container used in the method of detecting a target of the present invention, the second container is the second container used in the method of detecting a target of the present invention, the transfer unit is a unit for transferring the complex from the first container to the second container, and the detection unit is a unit for detecting the labeling substance in the step of detecting the complex. With respect to the detection apparatus of the present invention, the detection unit is preferably a unit for detecting the reaction of the detection reagent in the complex detection step.

The detection apparatus of the present invention is characterized by including the first container, the second container, the transfer unit, and the detection unit and being used for the detection method of the present invention, and other configurations are not limited at all.

The first container and the second container are, for example, the same as those described in the detection method of the present invention.

In the detection apparatus of the present invention, the first container and the second container may be, for example, components that are preliminarily provided in the detection apparatus or detachable components that can be attached at the time of using and can be detached after use.

The transfer unit is not particularly limited, and any transfer unit can be employed as long as the complex is transferred from the first container to the second container, for example. The transfer unit may be, for example, a sucking-and-discharging unit and a transfer control unit.

The sucking-and-discharging unit can be any unit as long as it sucks the complex from the first container and discharges the complex to the second container, for example. Preferably, the sucking-and-discharging unit is provided with a pressure control unit capable of decompressing and compressing and a tubular member; and the tubular member is in communication with the pressure control unit, for example. Examples of the pressure control unit include a pipetter, a plunger, a pump, an aspirator, a compression pump, and a decompression pump. Examples of the tubular member include a syringe, a tube, a nozzle, and a chip. The sucking-and-discharging unit may further be provided with a waste tank, for example. Specifically, the waste tank may be in communication with the tubular member.

The transfer control unit may be, for example, a unit for controlling the transfer of the tubular member in the sucking-and-discharging unit; a unit for controlling the transfer of the first container and the second container; or a unit for controlling the transfer of the tubular member, the first container, and the second container.

In the case where the transfer control unit is a unit for controlling the transfer of the tubular member, the complex can be transferred as follows. The transfer control unit can control the transfer of the tubular member in the axial direction thereof (for example, transfer in the up and down direction) and the transfer of the tubular member in the planar direction perpendicular to the axial direction (for example, transfer in the back and forth direction and the right and left direction), for example. In this case, for example, after the tubular member is arranged at the upper side of the first container, the tubular member is transferred downwardly and the tip of the tubular member is inserted into the first container. Then, after the complex in the first container is sucked from the tip of the tubular member using the sucking-and-discharging unit, the tubular member is returned to the upper side of the first container. Next, after the tubular member is arranged at the upper side of the second container by transferring it in the planar direction, the tubular member is transferred downwardly and the tip of the tubular member is inserted into the second container. Then, after the complex is discharged from the tip of the tubular member to the second container using the sucking-and-discharging unit, the tubular member is returned to the upper side of the second container. In this manner, the complex can be transferred from the first container to the second container.

Further, in the case where the transfer control unit is a unit for controlling the transfer of the tubular member and the transfer of the first container and the second container, the complex can be transferred as follows. The transfer control unit can control the transfer of the tubular member in the axial direction and the transfer of the containers in the planar direction perpendicular to the axial direction, for example. In this case, first, the first container is transferred in the planar direction to arrange it at the lower side of the tubular member. Then, in the same manner as described above, the tubular member is transferred downwardly and the tip of the tubular member is inserted into the first container. After the complex is sucked, the tubular member is returned to the upper side of the first container. Next, the first container and the second container are transferred in the planar direction to arrange the second container at the lower side of the tubular member. Then, in the same manner as described above, the tubular member is transferred downwardly and the tip of the tubular member is inserted into the second container. After the complex is discharged, the tubular member is returned to the upper side of the second container. In this manner, the complex can be transferred from the first container to the second container. In this case, preferably, the detection apparatus of the present invention includes a turn table to which the first container and the second container can be attached, and the turn table is rotated by the transfer control unit, for example.

The transfer unit may be, for example, an electrophoresis unit. The electrophoresis unit can be any unit as long as the complex is electrophoresed from the first container to the second container, for example. In this case, for example, preferably, the first container is in communication with the second container via a channel, and more preferably, two regions that are located far from each other in the flow direction in the channel are the first container (first chamber) and the second container (second chamber). An example of such a device includes the aforementioned uTAS. Preferably, the first container, the second container, and the channel contain a migration solution containing a migration agent, for example. Preferably, the electrophoresis unit is provided with at least two electrodes and a voltage application unit for applying a voltage between the electrodes, for example. Preferably, the electrodes are arranged on the first container and the second container respectively. Examples of the material of the electrode include stainless steel, platinum, and gold. Preferably, the voltage application unit is provided with a voltage device, a wire for connecting the electrodes and the voltage device, and the like, for example. By applying a voltage between the first container and the second container with the voltage application unit, the complex can be transferred from the first container to the second container by an electrophoresis.

The transfer unit may be, for example, the magnetic body and a transfer control unit. The magnetic body can be any magnetic body as long as it has, for example, a magnetic force, and an example thereof includes a magnet. The transfer control unit may be, for example, a unit for controlling the transfer of the magnetic body; a unit for controlling the transfer of the first container and the second container; or a unit for controlling the transfer of the magnetic body, the first container, and the second container.

In the case where the transfer control unit is a unit for controlling the transfer of the magnetic body, for example, the complex can be transferred as follows. The transfer control unit can control the transfer of the magnetic body in the direction of bringing it close to the external wall of the first container and the external wall of the second container and the transfer of the magnetic body in the direction of bringing it away from the external wall of the first container and the external wall of the second container, for example. For example, the transfer control unit can control the transfer of the magnetic body from the first container toward the second container. Specifically, for example, in the case where the first container is in communication with the second container via the channel, the transfer control unit can control the transfer of the magnetic body from the external wall of the first container to the second container along the external wall of the channel. In this case, for example, by bringing the magnetic body close to the external wall of the first container, the complex in the first container is captured. Next, the magnetic body is transferred to the external wall of the second container along the channel direction, for example. Further, by bringing the magnetic body away from the external wall of the second container, the complex in the second container is released. In this manner, the complex can be transferred from the first container to the second container.

The detection unit is not particularly limited, and the detection unit can be any unit as long as it can detect the labeling substance. The detection unit can be selected appropriately depending on the type of labeling substance, for example. In the present invention, in the case where the labeling substance is a substance that can be detected alone, for example, being capable of detecting the labeling substance means, for example, being capable of detecting a signal that is caused due to the labeling substance. Further, in the case where the labeling substance is a substance that can be detected through reaction with a detection reagent, for example, being capable of detecting the labeling substance means, for example, being capable of detecting a signal that is caused due to the reaction of the labeling substance in the complex and the detection reagent by reacting the detection reagent capable of detecting a labeling substance and the complex. A specific example of the detection unit includes a detector including a light source portion, a light receiving portion, and the like. For example, the detector emits light with a specific wavelength from the light source portion to a target object to be measured and receives transmitted light, reflected light, or the like as a signal at the light receiving portion. Examples of the light source portion include a light-emitting diode (LED) and a semiconductor laser diode (LD). Examples of the light receiving portion include a photodiode, a photomultiplier tube, and a CCD image sensor.

The detection apparatus of the present invention may further include a computing unit. For example, from a signal that is detected by the detection unit, the computing unit can compute the intensity thereof. Specifically, for example, a transmittance, an absorbance, a reflectance, luminescence intensity, fluorescence intensity, and the like can be computed as the signal intensity from the light received at the light receiving portion in the detection unit, for example.

The detection apparatus of the present invention may further include a control unit for controlling the wavelength or the like of the light emitted from the light source portion, for example. The detection apparatus may be provided with input units such as a CPU, a memory, a keyboard, and a touch panel and output units such as a display and a printer, for example. Examples

Next, the present invention will be described in detail with the following Examples. However, the present invention is not limited to these Examples.

### [Example 1]

In this example, a fluorescent enzyme immunoassay was performed using antibody immobilized magnetic particles with physiological saline that does not contain a target as a sample.

### (1) Preparation of Antibody Immobilized Magnetic Particle

Anti-human luteinizing hormon (LH) antibody (product of Medix) was used as a first antibody and "Dynabeads® MyOne Tosylactivated" (trade name, product of Dynal Biotech) was used as a magnetic bead. According to the protocol for adding the magnetic bead, the first antibody was immobilized to the magnetic bead, and blocking treatment was applied thereto. In this manner, an antibody immobilized magnetic particle was prepared.

### (2) Preparation of Enzyme-Labeled Antibody

Anti-human luteinizing hormon (LH) antibody (product of Fitzgerald Industries International) was used as a second antibody and alkaline phosphatase (trade name, "Alkaline Phosphatase labeling kit-SH", product of Dojindo Laboratories) was used as a labeling substance. According to the protocol for adding the alkaline phosphatase, the second antibody was labeled with the alkaline phosphatase. In this manner, an enzyme-labeled antibody was prepared.

### (3) Preparation of Reagent

The antibody immobilized magnetic particles were mixed with the buffer solution 1 (pH 7.4) having the following composition so as to obtain the resultant mixture having a concentration of 0.5 mg/mL. In this manner, a particle solution was prepared. Further the enzyme-labeled antibodies were mixed with the buffer solution 2 (pH 7.4) having the following composition so as to obtain the resultant mixture having a concentration of 0.7 µg/mL. In this manner, a labeled antibody solution was prepared.

| (Composition of buffer solution 1) | |
|---|---|
| 50 mmol/L | Tris |
| 150 mmol/L | NaCl |
| 0.1 w/v% | BSA |
| 0.05 w/v% | NaN₃ |

| (Composition of buffer solution 2) | |
|---|---|
| 50 mmol/L | Tris |
| 150 mmol/L | NaCl |
| 0.1 w/v% | BSA |
| 0.05 w/v% | NaN₃ |
| 0.025 mmol/L | ZnCl₂ |
| 1 mmol/L | MgCl₂ |

### (4) Measurement of Fluorescence

50 µL of the sample (physiological saline) and 50 µL of the labeled antibody solution were added to a first tube with a capacity of 1.5 mL ("Cat.No./MCT-150-L-C", product ofAxygen) and mixed, and the resultant mixture was incubated at 37°C for 5 minutes. 50 µL of the particle solution was further added to the first tube and suspended, and the resultant mixture was incubated at 37°C for 5 minutes. Next, the magnetic particles were captured using a magnet from the outside of the first tube, and the supernatant in the first tube was removed in this state. Then, after 500 µL of the washing liquid (pH 7.4) having the following composition was added to the first tube, the magnet was separated from the first tube, and the magnetic particles were washed in the first tube by suspending the magnetic particles captured in the washing liquid. After washing, in the state where the magnetic particles were captured in the same manner as described above, the washing liquid was removed from the first tube. The washing treatment and the removal treatment of the washing liquid were repeated twice in total. Further, 500 µL of the washing liquid was added again to the first tube, the magnetic particles captured were suspended, and the whole suspension was transferred to a new second tube with a capacity of 1.5mL ("Cat.No./MCT-150-L-C", product of Axygen). Then, in the same manner as described above, the magnetic particles were captured, and the supernatant in the second tube was removed in this state. 500 µL of the substrate liquid (pH 9.6) having the following composition containing 4-methylumbelliferyl phosphate was added to the second tube to react with the solution therein at 37°C for 10 minutes. The magnetic particles were captured, and the solution in the second tube was recovered in this state in the same manner as described above. The solution recovered was transferred to a measurement container, 50 µL of a sodium hydroxide solution having a concentration of 2 mol/L was added and the reaction was suspended. Then, the fluorescence intensity of 4-methylumbelliferyl generated in the resultant solution was measured at an excitation wavelength of 365 nm and a detection wavelength of 450 nm. The measurement value of the fluorescence intensity was adjusted such that the fluorescence intensity of the substrate liquid showed 0. The measurement was performed 5 times, and the average value of the fluorescence intensity was calculated.

| (Composition of washing liquid) | |
|---|---|
| 50 mmol/L | Tris |
| 150 mmol/L | NaCl |
| 0.05 w/v% | Tween20® |
| 0.05 w/v% | NaN₃ |

| (Composition of substrate liquid) | |
|---|---|
| 600 mmol/L | 2-EAE |
| 0.6 mmol/L | 4-methylumbelliferyl phosphate (4-MUP) |
| 0.5 w/v% | Fructose |
| 0.09 w/v% | NaN₃ |

### [Comparative Example 1]

In this example, the fluorescence intensity was measured in the same manner as in example 1 except that an enzyme that is the labeling substance and the substrate were reacted in the first tube without transferring the suspension to the second tube. Specifically, after the magnetic particles were washed in the first tube, 500µL of the washing liquid was added again to the first tube, the magnetic particles captured were suspended, and the supernatant was removed. Then, the substrate liquid was added to the first tube to react with the solution therein, the resultant solution recovered was transferred to a measurement container, and the fluorescence intensity of the resultant solution was measured after the reaction was suspended.

In the following Table 1, the average values of the fluorescence intensity of the solutions obtained in example 1 and comparative example 1 are summarized. In this example, as described above, physiological saline that does not contain a target was used as a sample. Therefore, if the average value is low, it means that an increase in fluorescence intensity, i.e., an increase in background that causes a nonspecific adsorption to a substance other than the target is suppressed. As summarized in the following Table 1, the average value of the fluorescence intensity of the solution obtained in Comparative example 1 exceeded 1,000 whereas the average value of the fluorescence intensity of the solution obtained in example 1 was less than 0, which was the detection limit. In this manner, it was found out that the effect of the nonspecific adsorption of the labeled antibody to the first tube can be avoided and an increase in background can be suppressed by reacting an enzyme that is the labeling substance and a substrate not in the first tube to which the labeled antibody was added but in a new second tube.

**(Table 1)**

| Average value of fluorescence intensity | |
|---|---|
| | |
| Example 1 | -68 |
| Comparative Example 1 | 1080 |

### [Example 2]

In this example, a fluorescent enzyme immunoassay was performed using streptavidin immobilized magnetic particles and biotin-conjugated antibodies with physiological saline that does not contain a target as a sample.

### (1) Preparation of Streptavidin Immobilized Magnetic Particle

"Dynabeads® MyOne Streptavidin T1" (trade name, product of Dynal Biotech) was used as a magnetic bead. According to the protocol for adding the magnetic bead, blocking treatment was applied to the magnetic bead using BSA. In this manner, a streptavidin immobilized magnetic particle was prepared.

### (2) Preparation of Biotin-Conjugated Antibody

Anti-human luteinizing hormon (LH) antibody (product of MEDIX) was used as a first antibody. By the use of Biotin labeling kit-SH (product of Dojindo Laboratories), biotin was conjugated to the first antibody according to the protocol. In this manner, a biotin-conjugated antibody was prepared.

### (3) Preparation of Reagent

The streptavidin immobilized magnetic particles were mixed with the buffer solution 1 so as to obtain the resultant mixture having a concentration of 0.5 mg/mL. In this manner, a particle solution was prepared. Further, the biotin-conjugated antibodies were mixed with the buffer solution 1 so as to obtain the resultant mixture having a concentration of 1 µg/mL. In this manner, a biotin-conjugated antibody solution was prepared. Further, in the same manner as in example 1, the labeled antibody solution was prepared.

### (4) Measurement of Fluorescence

The average value of the fluorescence intensity was calculated in the same manner as in example 1 except for the following differences: 50 µL of the biotin-conjugated antibody solution was further added to the first tube in addition to the sample (physiological saline) and the labeled antibody solution and mixed, and the resultant mixture was incubated at 37°C for 5 minutes.

### [Comparative Example 2]

In this example, the average value of the fluorescence intensity was calculated in the same manner as in example 2 except that an enzyme that is the labeling substance and the substrate were reacted in the first tube without transferring the suspension to the second tube. Specifically, after the magnetic particles were washed in the first tube, 500µL of the washing liquid was added again to the first tube, the magnetic particles captured were suspended, and the supernatant was removed. Then, the substrate liquid was added to the first tube to react with the solution therein, the resultant solution recovered was transferred to a measurement container, and the fluorescence intensity of the resultant solution was measured after the reaction was suspended.

In the following Table 2, the average values of the fluorescence intensity of the solutions obtained in example 2 and comparative example 2 are summarized. As summarized in the following Table 2, the average value of the fluorescence intensity of the solution obtained in comparative example 2 exceeded 1,600 whereas the average value of the fluorescence intensity of the solution obtained in example 2 was less than 0, which was the detection limit. In this manner, it was found out that the effect of the nonspecific adsorption of the labeled antibody to the first tube can be avoided and an increase in background can be suppressed by reacting an enzyme that is the labeling substance and a substrate not in the first tube to which the labeled antibody was added but in a new second tube.

**(Table 2)**

| | Average value of fluorescence intensity |
|---|---|
| Example 2 | -36 |
| Comparative Example 2 | 1602 |

### [Example 3]

In this example, a fluorescent enzyme immunoassay was performed in the same manner as in example 2 except that the buffer solution 3 (pH 7.4) having the following composition was used instead of the physiological saline as the sample.

| (Composition of buffer solution 3) | |
|---|---|
| 50 mmol/L | Tris |
| 150 mmol/L | NaCl |
| 0.05 w/v% | BSA |
| 0.05 w/v% | NaN₃ |

### [Comparative Example 3-1]

In this example, the average value of the fluorescence intensity was calculated in the same manner as in example 3 except that an enzyme that is the labeling substance and the substrate were reacted in the first tube without transferring the suspension to the second tube.

### [Comparative Example 3-2]

In this example, the average value of the fluorescence intensity was calculated in the same manner as in Comparative Example 3-1 except that a low adsorption tube (trade name, "A.150MPC", product of ASSIST) was used as the first tube.

### [Comparative Example 3-3]

In this example, the average value of the fluorescence intensity was calculated in the same manner as in comparative example 3-1 except that a low adsorption tube (trade name, "Protein LoBind tube", product of Eppendorf) was used as the first tube.

### [Comparative Example 3-4]

In this example, the average value of the fluorescence intensity was calculated in the same manner as in comparative example 3-1 except that a low adsorption tube (trade name, "BioPure tube", product of Eppendorf) was used as the first tube.

In the following Table 3, the average values of the fluorescence intensity of the solutions obtained in example 3 and comparative examples 3-1 to 3-4 are summarized. In this Example, as described above, physiological saline that does not contain a target was used as a sample. As summarized in the following Table 3, the average values of the fluorescence intensity of the solutions obtained in comparative examples 3-1 to 3-4 exceeded 300 whereas the average value of the fluorescence intensity of the solution obtained in example 3 was less than 150. In this manner, it was found out that the effect of the nonspecific adsorption of the labeled antibody to the first tube can be avoided and an increase in background can be suppressed by reacting an enzyme that is the labeling substance and a substrate not in the first tube to which the labeled antibody was added but in a new second tube.

**(Table 3)**

| | Average value of fluorescence intensity |
|---|---|
| Example 3 | 142 |
| Comparative Example 3-1 | 354 |
| Comparative Example 3-2 | 524 |
| Comparative Example 3-3 | 510 |
| Comparative Example 3-4 | 545 |

### [Example 4]

In this example, an anti human insulin antibody (product of Roche) was used as a first antibody and an anti human insulin antibody (product of Roche) was used as a second antibody. The concentration of the antibody immobilized magnetic particle in the particle solution was 0.3 mg/mL and the concentration of the enzyme labeled antibody in the labeled antibody solution was 1 µg/mL. Further, the amount of the sample (physiological saline) added to the first tube was 20 µL, the amount of the labeled antibody solution added to the first tube was 100 µL, and the amount of the particle solution added to the first tube was 100 µL; and washing treatment was performed once. Except for these, a fluorescent enzyme immunoassay was performed in the same manner as in example 1.

### [Comparative Example 4]

In this example, after the magnetic particles were washed in the first tube, the supernatant was removed, and the substrate liquid of example 1 was further added to the first tube and suspended. Then, right after addition of the substrate liquid, the whole mixture was transferred from the first tube to a new second tube with a capacity of 1.5 mL ("Cat.No./MCT-150-L-C", product ofAxygen) and was reacted at 37°C for 10 minutes. Except for these, the average value of the fluorescence intensity was calculated in the same manner as in Example 4.

In the following Table 4, the average values of the fluorescence intensity of the solutions obtained in example 4 and comparative example 4 are summarized. As summarized in the following Table 4, the average value of the fluorescence intensity of the solution obtained in comparative example 4 exceeded 7,000 whereas the average value of the fluorescence intensity of the solution obtained in example 4 was less than 2,000. This showed that when the labeling substance was detected in a state where the magnetic particles and the substrate that is the detection reagent coexisted in the second tube after the magnetic particles were transferred from the first tube to the second tube, an increase in background was further suppressed as compared to the case in which the magnetic particles and the substrate coexisted in the first tube and the mixture was then transferred to the second tube. From these results, it was considered that an increase in background in comparative example 4 was caused by the reaction between the unreacted labeled antibody adsorbed to the first tube and the substrate.

**(Table 4)**

| | Average value of fluorescence intensity |
|---|---|
| Example 4 | 1911 |
| Comparative Example 4 | 7452 |

### [Example 5]

In this example, the following human insulin-added sample and human insulin-non-added sample were used, an anti human insulin antibody (product of Roche) was used as a first antibody, and an anti human insulin antibody (product of Roche) was used as a second antibody. The concentration of the antibody immobilized magnetic particle in the particle solution was 0.3 mg/mL and the concentration of the enzyme labeled antibody in the labeled antibody solution was 1 µg/mL. Further, the amount of the labeled antibody solution added to the first tube was 100 µL and the amount of the particle solution added to the first tube was 100 µL; and measurement was performed three times. Except for these, a fluorescent enzyme immunoassay was performed in the same manner as in example 1.

As the samples, a sample that was prepared by adding human insulin (trade name, "Insulin Human recombinant, Cat.No.30-AI51") to the buffer solution 4 (pH 7.4) having the following composition so as to obtain the resultant mixture having a concentration of 0.5 µIU/mL and a sample (0 µIU/mL) that was prepared without adding the human insulin were used.

| (Composition of buffer solution 4) | |
|---|---|
| 100 mmol/L | Tris |
| 0.1 w/v% | BSA |
| 0.5 w/v% | Fructose |
| 0.1 w/v% | NaN₃ |

### [Comparative Example 5]

In this example, the average value of the fluorescence intensity was calculated in the same manner as in example 5 except that an enzyme that is the labeling substance and the substrate were reacted in the first tube without transferring the suspension to the second tube.

In the following Table 5, the average values of the fluorescence intensity of the solutions obtained in example 5 and comparative example 5 are summarized. As summarized in the following Table 5, with respect to the sample to which insulin was not added, the average value of the fluorescence intensity of the solution obtained in comparative example 5 exceeded 1,000 whereas the average value of the fluorescence intensity of the solution obtained in example 5 was less than 20. Further, with respect to the sample having an insulin concentration of 0.5 µIU/mL to which insulin that was a target was added, the average value of the fluorescence intensity of the solution obtained in comparative example 5 exceeded 5,000 whereas the average value of the fluorescence intensity of the solution obtained in example 5 was less than 2,000. This showed that, even when the sample contains a target, when the labeling substance was detected in a state where the magnetic particles and the substrate that is the detection reagent coexisted in the second tube after the magnetic particles were transferred from the first tube to the second tube, an increase in background was suppressed.

**(Table 5)**

| | Insulin concentration (µIU/mL) | |
|---|---|---|
| | 0 | 0.5 |
| Example 5 | 13 | 1765 |
| Comparative Example 5 | 1706 | 5797 |

### [Example 6]

In this example, a fluorescent enzyme immunoassay was performed with the sample containing human insulin by performing the B/F separation after the complex was transferred to the second tube.

### (1) Preparation of Antibody Immobilized Magnetic Particle

An antibody immobilized magnetic particle was prepared in the same manner as in example 1 except that an anti human insulin antibody (product of Roche) was used as a first antibody.

### (2) Preparation of Enzyme Labeled Antibody

An enzyme labeled antibody was prepared in the same manner as in Example 1 except that a human insulin antibody (product of Roche) was used as a second antibody.

### (3) Preparation of Reagent

The antibody immobilized magnetic particles were mixed with the buffer solution 1 so as to obtain the resultant mixture having a concentration of 0.3 mg/mL. In this manner, a particle solution was prepared. Further, the enzyme labeled antibody was mixed with the buffer solution 2 so as to obtain the resultant mixture having a concentration of 1 µg/mL. In this manner, a labeled antibody solution was prepared.

### (4) Preparation of Sample

As the sample, a sample that was prepared by adding human insulin (trade name, "Insulin Human recombinant, Cat.No.30-AI51") to the buffer solution 4 (pH 7.4) so as to obtain the resultant mixture having a concentration of 0.5 µIU/mL was used.

### (5) Measurement of Fluorescence

50 µL of the sample and 100 µL of the labeled antibody solution were added to a first tube with a capacity of 1.5 mL ("Cat.No./MCT-150-L-C", product ofAxygen) and mixed, and the resultant was incubated at 37°C for 5 minutes. 100 µL of the particle solution was further added to the first tube and suspended, and the resultant mixture was incubated at 37°C for 5 minutes. Next, the whole suspension was transferred to a new second tube with a capacity of 1.5 mL ("Cat.No./MCT-150-L-C", product ofAxygen). Then, the supernatant in the second tube was removed in the state where the magnetic particles were captured using a magnet from the outside of the second tube. The magnetic particles were washed in the second tube by adding 500 µL of the washing liquid (pH 7.4) to the second tube and suspending the captured magnetic particles in the washing liquid. After washing, in the state where the magnetic particles were captured in the same manner as described above, the washing liquid was removed from the second tube. The washing treatment and the removal treatment of the washing liquid were repeated twice in total. 500 µL of the substrate liquid (pH 9.6) containing 4-methylumbelliferyl phosphate was added to the second tube to react with the solution therein at 37°C for 10 minutes. The resultant solution in the second tube was recovered in the state where the magnetic particles were captured in the same manner as described above. The solution recovered was transferred to a measurement container, 50 µL of a sodium hydroxide solution having a concentration of 2 mol/L was added and the reaction was suspended. Then, the fluorescence intensity of 4-methylumbelliferyl generated in the resultant solution was measured at an excitation wavelength of 365 nm and a detection wavelength of 450 nm. The measurement value of the fluorescence intensity was adjusted such that the fluorescence intensity of the substrate liquid showed 0. The measurement was performed 3 times, and the average value of the fluorescence intensity was calculated.

### [Comparative Example 6]

In this example, the average value of the fluorescence intensity was calculated in the same manner as in example 6 except that an enzyme that is the labeling substance and the substrate were reacted in the first tube without transferring the suspension to the second tube.

In the following Table 6, the average values of the fluorescence intensity of the solutions obtained in example 6 and comparative example 6 are summarized. As summarized in the following Table 6, the average value of the fluorescence intensity of the solution obtained in comparative example 6 exceeded 400 whereas the average value of the fluorescence intensity of the solution obtained in example 6 was less than 100. This showed that when the labeling substance was detected in a state where the magnetic particles and the substrate that is the detection reagent coexisted in the second tube after the magnetic particles were transferred from the first tube to the second tube, an increase in background was suppressed. These results showed that the signal that may be caused by the reaction between the unreacted labeled antibody adsorbed to the first tube and the substrate was suppressed even when the complex was transferred to the second tube before the B/F separation.

**(Table 6)**

| | Average value of fluorescence intensity |
|---|---|
| Example 6 | 95 |
| Comparative Example 6 | 419 |

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A method of detecting a target using a first target-binding substance immobilized at a carrier and a second target-binding substance labeled with a labeling substance, comprising the steps of:
reacting a target, the first target-binding substance, and the second target-binding substance to form a complex of the target, the first target-binding substance, and the second target-binding substance in a first container;
transferring the complex from the first container to a second container; and
detecting the complex by detecting the labeling substance in the second container.

2. The method according to claim 1, wherein
the step of detecting the complex comprises reacting a detection reagent capable of detecting the labeling substance with the complex and detecting the labeling substance through detection of the reaction in the second container.

3. The method according to claim 1 or 2, further comprising the step of:
removing a liberated second target-binding substance after the step of forming the complex, wherein the step of removing is performed before and/or after the step of transferring the complex from the first container to a second container.

4. The method according to claim 3, wherein
the step of removing comprises a step of washing the complex.

5. The method according to any one of the preceding claims, wherein each of the first target-binding substance and the second target-binding substance is at least one selected from the group consisting of antigens, antibodies, and nucleic acids.

6. The method according to any one of the preceding claims, wherein each of the first target-binding substance and the second target-binding substance is an antibody.

7. The method according to any one of claims 1 to 5, wherein
each of the first target-binding substance and the second target-binding substance is at least one selected from the group consisting of biotin, avidin, streptavidin, proteins, peptide, sugar chains, lipid, hormones, ligand, and receptors.

8. The method according to any one of claims 1 to 7, wherein
the carrier is at least one selected from the group consisting of magnetic particles, latex particles, metal colloid particles, polymer particles, and glass particles.

9. The method according to any one of claims 1 to 8, wherein
the labeling substance is an enzyme.

10. The method according to claim 9, wherein the detection reagent is a substrate of the enzyme.

11. The method according to any one of claims 1 to 10, wherein the target is selected from the group consisting of pathogens, biogenic substances, endocrine-disrupting chemicals, allergic substances in foods, and agricultural chemicals.

12. A method of suppressing an increase in background in detection of a target using a first target-binding substance immobilized at a carrier and a second target-binding substance labeled with a labeling substance, comprising the steps of:
reacting a target, the first target-binding substance, and the second target-binding substance to form a complex of the target, the first target-binding substance, and the second target-binding substance in a first container;
transferring the complex from the first container to a second container; and
detecting the complex by detecting the labeling substance in the second container.

13. The method according to claim 12, wherein
the step of detecting the complex comprises reacting a detection reagent capable of detecting the labeling substance with the complex and detecting the labeling substance through detection of the reaction in the second container.

14. A detection apparatus for use in the method of detecting a target according to any one of claims 1 to 11, comprising:
a first container;
a second container;
a transfer unit; and
a detection unit, wherein
the first container is the first container according to any one of claims 1 to 11, the second container is the second container according to any one of claims 1 to 11,
the transfer unit is a unit for transferring the complex from the first container to the second container, and
the detection unit is a unit for detecting the labeling substance in the step of detecting the complex.
